# EUROPEAN PATENT APPLICATION

(11) **EP 1 199 078 A1**
(43) Date of publication of application: **24.04.2002**
(21) Application number: 00937186.5
(22) Date of filing: 08.06.2000
(51) Int. Cl.: A61K 31/435, A61P 29/00, C07D 221/04, C07D 221/20

(54) **ANALGESICS**

(30) Priority: 09.06.1999 JP 16255899
(71) Applicant: ONO PHARMACEUTICAL CO., LTD., Osaka-shi, Osaka 541-8526 (JP)
(72) Inventor: NAKA, Masao, Minase Res. Ins., Ono Pharm. Co. Ltd, Mishima-gun, Osaka 618-8585 (JP); KOBAYASHI, Kaoru, Minase Res. Ins., Ono Parm. Co., Mishima-gun, Osaka 618-8585 (JP)
(74) Representative: Henkel, Feiler, Hänzel
(86) International application number: JP0003721
(87) International publication number: WO0074678

(57) **Abstract**

The prevention and/or treatment of pain, hyperalgesia and allodynia according to claim 1 which comprise, as an active ingredient, a condensed piperidine compound represented by formula (I), a non-toxic salt thereof, wherein all the symbols have the same meanings as defined in the specification.

The compound of formula (I) of the present invention has analgetic effect in lower doses.

## Description

### TECHNICAL FIELD

The invention relates to an analgesic which comprise, as an active ingredient, a condensed piperidine compound represented by formula (I), a non-toxic salt thereof or a hydrate thereof, wherein all the symbols have the same meanings as defined hereinafter.

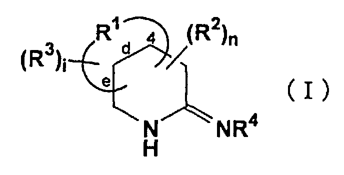

### BACKGROUND

Pain is not only a warning response for the life maintenance against external noxious stimuli and pathological situations in the body but also pain brings harmful stage to the body by itself, that is, pain has binary character. Under physiological condition, pain is produced as a warning response against a noxious stimuli which activate nociceptor localized in the peripheral tissue followed by the liberation of algogenic substances. Under pathological conditions such as inflammation in peripheral tissues and tissue damage, however, a persistence production of algogenic substances can continuously activate nociceptor, which results in lowering the threshold for noxious stimulus of heat, mechanical and chemical stimuli. This situation brings "hyperalgesia" stage.

Non-noxious stimulus such as touch or pressure is transmitted from peripheral non-nociceptor to central nerve system, but the stimulus does not cause pain under physiological condition. On the other hand, under pathological condition light touch and cold stimulation can cause pain. This symptom is so called "allodynia". Allodynia is associated with pain caused by peripheral nerve injury (e.g. causalgia, sympathetic reflex dystrophy), central nerve injury (e.g. phantom limb pain, post-zoster neuralgia (post-herpetic pain), thalamic pain, pain after spinal cord injury), diabetic neuropathy or nerve invasive cancer.

On one hand, the discovery that microphages, one of immuno-competent cells, produced a large quantity of nitrate led to the discovery that nitric oxide (NO) was biologically synthesized in the body (*Proc. Natl. Acad. Sci., U.S.A.,* Vol. 82, pp.7738-7742 (1985) and *J Immunol.*, Vol. 138, pp.550-565(1987)). In the field of circulatory organs, a substance having vasodilating action released from vascular endothelial cells was discovered and named the endothelial cell-derived relaxing factor (EDRF). It was found later that the substance of EDRF was NO (*Nature*, Vol. 327, pp.524-526 (1987)).

NO, which has recently been found being produced in the body, is synthesized from L-arginine as a substrate through the following pathway by an enzymatic action of nitric oxide synthase (hereinafter abbreviated as NOS).

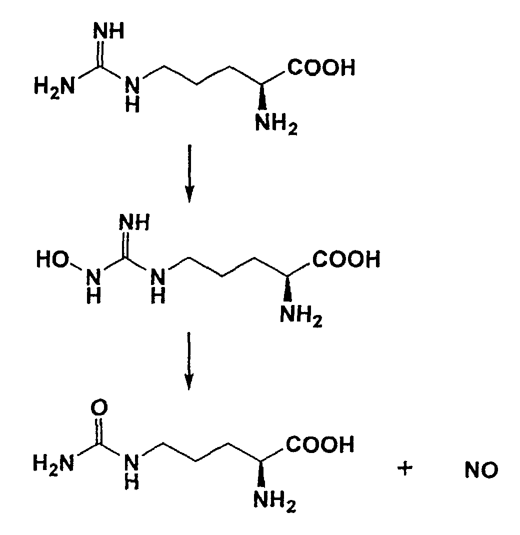

NOS includes at least a constitutive isozyme (endothelial type and neuronal type) and an inducible isozyme. Endothelial NOS mainly exists in vascular endothelial cells, and its activity is regulated by an intracellular calcium concentration. Neuronal NOS exists in central nerve cells, peripheral nerve cells, islet β cells, gastrointestinal nerves, the medulla of the suprarenal glands, renal macula densa, etc. Its activity is also regulated by the intracellular calcium concentration as well as the endothelial type NOS.

The constitutive NOS (inclusive of endothelial type and neuronal type, hereinafter abbreviated as c-NOS) exist in the cells and the amount of enzymes is hardly changed under physiological conditions. On the other hand, the inducible NOS (hereinafter abbreviated as i-NOS) is not present in cells under physiological condition, however, it can be induced by the stimulation of endotoxin and/or various cytokines in hepatocytes, neutrophil, macrophages, smooth muscle cells, fibroblasts, mesangium cells, gastrointestinal epithelium cells, islet β cells, vascular smooth cells, gliocyte, etc.

NO synthesized by NOS exerts a wide variety of actions, such as vascular relaxation, inhibition of platelet aggregation and adherence, inhibition of leukocyte migration and adherence, inhibition of sympathetic activity, anti-tumor action, bactericidal action and NO itself can plays as a neurotransmitter. Moreover, NO might be involved in the pathogenesis in endotoxin- or cytokine- induced hypotention, ischemic cerebral cell disturbances, induction of autoimmune diseases, insulin dependent diabetes mellitus, arthritis, induction of post-transplantation tissue disturbances, and graft rejection, so on.

It is conceivable to use NO synthase inhibitor (NOS inhibitor) to analyze the physiological role of NO, and more importantly, it would be useful as a possible therapeutic agent in the treatment of shocks or ischemic diseases. Therefore, a considerable effort has been made to develop NOS inhibitors.

For example, arginine analogues, such as N ω-monomethyl-L-arginine (L-NMMA), N ω-nitro-L-arginine (L-NNA), N ω-iminoethylornithine (L-NIO) are known as a substrate competitive agent. Known cofactor competitive inhibitors include diphenylene iodonium (DPI), di-2-thienyl iodonium (DTI), and calcineurin. Known inhibitor of gene transcriptional induction include corticosteroid, TGFβ, IL-4 and IL-10.

On one hand, it is known that NO behaves as a neurotransmitter to exert nociceptive response in spinal cord (*pain*, Vol. 52, pp.127-136 (1993)) and NO plays a role as chemical mediator in a model of the thermal hyperalgesia in rats (*Neuroscience*, Vol. 5, pp.7-10 (1992)). Moreover, it is reported that treatment of 50 µmol/kg of L-NAME showed effect on allodynia model in rats (*Neuroreport*, Vol. 9, pp.367-372 (1998)).

It is known that NOS inhibitor is useful for the treatment of various diseases where NO might be involved, because it can inhibit NO synthesis in the body. Wherein it is known that specific compounds, such as L-NAME, have an action to relieve pain.

However, they are not enough for the treatment of pain because its effect is weak. Therefore, it is necessary to develop a novel NOS inhibitor with having a potent analgetic effect.

### DISCLOSURE OF THE INVENTION

As described previously, it is known L-NAME, which is NOS inhibitor, is useful for hyperalgesia and allodynia, but the effect is week.

The inventors have found that the compounds represented by formula (I), which were disclosed as NOS inhibitors in EP870763A specification, are much potent inhibitors against NOS. In fact, it has been found that the compounds are useful for prevention and/or treatment of pain, hyperalgesia and allodynia in a low dose range. The present invention has been completed based on this finding.

That is, the invention relates to prevention and/or treatment of pain as defined hereinafter.
1) a prevention and/or treatment of pain which comprise, as an active ingredient, a condensed piperidine compound represented by formula (I), an acid addition salt thereof or a hydrate thereof: 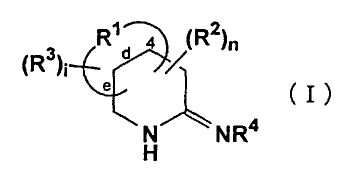 wherein -R1- represents a 3- or 4-membred carbocyclic ring together with the carbon atom or atoms to which it is bonded, the carbocyclic ring being condensed to side d or e of the piperidine ring or bonded to the 4-position of the piperidine ring through a spiro-bond;
   R2 represents a C1-6 alkyl group;
   R3 represents a C1-6 alkyl group, a C2-6 alkenyl group, a C2-6 alkynyl group or halogen atom;
   R4 represents a hydrogen atom, an amino-C1-4 alkyl group or a carbocyclic ring-C1-4 alkyl group which may be substituted with an amino-C1-4 alkyl group;
   i represents 0 or an integer of 1 to 3;
   n represents 0 or an integer of 1 to 3; and
   the plural R2's or R3's are the same or different.
2) the prevention and/or treatment of pain described in the above 1 which comprise, as an active ingredient, a condensed piperidine compound represented by formula (IA), an acid addition salt thereof or a hydrate thereof: 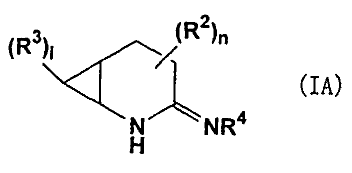 wherein all the symbols have the same meanings as defined in the above 1.
3) the prevention and/or treatment of pain described in the above 1 which comprise, as an active ingredient, a condensed piperidine compound represented by formula (I), an acid addition salt thereof or a hydrate thereof, wherein i is zero.
4) the prevention and/or treatment of pain described in the above 1 which comprise, as an active ingredient, a condensed piperidine compound represented by formula (I), an acid addition salt thereof or a hydrate thereof, wherein i is one, two or three, and R3 is selected from the group consisting of a C1-6 alkyl group, a C2-6 alkenyl group, and C2-6 alkynyl group.
5) the prevention and/or treatment of pain described in the above 1 which comprise, as an active ingredient, a condensed piperidine compound represented by formula (I), an acid addition salt thereof or a hydrate thereof, wherein i is one, two or three, and at least one of the plural R3's is a halogen atom.
6) the prevention and/or treatment of pain described in the above 1 which comprise, as an active ingredient, a condensed piperidine compound represented by formula (IA-1), an acid addition salt thereof or a hydrate thereof: 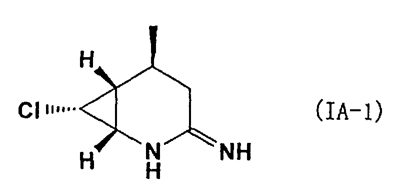
7) the prevention and/or treatment described in any one of the above 1 to 6 of pain, which is hyperalgesia.
8) the prevention and/or treatment described in any one of the above 1 to 6 of pain, which is allodynia.
9) the prevention and/or treatment described in any one of the above 1 to 8 of pain, which is allodynia with pain caused by peripheral or central nerve injury, diabetic neuropathy or nerve invasive cancer.

The compound used in the present invention may be administered in the form of the acid addition salt thereof.

It is desirable for the acid addition salt be non-toxic and water-soluble. Suitable acid addition salts include inorganic salts (e.g., hydrochloride, hydrobromide, hydroiodide, sulfate, phosphate, nitrate); and/organic acid salts (e.g., acetate, lactate, tartrate, oxalate, fumarate, maleate, citrate, benzoate, methanesulfonate, ethanesulfonate, benzenesulfonate, toluenesulfonate, isethionate, glucuronate, gluconate). Hydrochloride is preferable.

The compound of formula (I) of the present invention or a salt thereof can be converted to its hydrate in known manner.

### [Process for preparing the compound of the invention]

The compound of formula (I) of the present invention may be preperated by methods described in EP870763A.

### [Pharmacological activities of the compound of the invention]

The compound of formula (I) of the present invention has a potent NOS inhibitory activity. As described hereinafter, the compound of formula (IA-1) of the present invention showed analgetic effects in the formalin test and Bennett model in rats.

### [Toxicity]

The toxicity of the compounds of the present invention is very low and therefore the compounds may be considered safe for pharmaceutical use. For example, maximum tolerated dose with an intravenous injection is 30mg/kg in mice.

### [Application to Pharmaceutical Compositions]

The compound of formula (I) of the present invention, an acid addition salt thereof or a hydrate thereof has analgetic effect and is useful for prevention and/or treatment of pain, hyperalgesia or allodynia. It is useful for the prevention and/or the treatment of allodynia associated with pain caused by peripheral nerve injury (e.g. causalgia, sympathetic reflex dystrophy), central nerve injury (e.g., phantom limb pain, post-zoster neuralgia (post-herpetic pain), thalamic pain, pain after spinal cord injury), and diabetic neuropathy or nerve invasive cancer.

For the purpose above described, the compounds of formulae (I) of the present invention, acid addition salts or hydrates thereof may be normally administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered in the form of, for example, solid forms for oral administration, liquid forms for oral administration, injections, liniments or suppositories for parenteral administration.

Solid forms for oral administration include compressed tablets, pills, capsules, dispersible powders, and granules. Capsules include hard capsules and soft capsules.

In such solid forms, one or more of the active compound(s) may be admixed with vehicles (such as lactose, mannitol, glucose, microcrystalline cellulose, starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include pharmaceutically acceptable solutions, suspensions and emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol, e.g. ethanol, or a mixture thereof.

Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms such as freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known per se. Sprays may comprise additional substances other than diluents, such as stabilizing agents (such as sodium sulfate), isotonic buffers (such as sodium chloride, sodium citrate or citric acid). For preparation of such sprays, for example, the method described in the United States Patent No. 2,868,691 or 3,095,355 may be used.

### Brief Description of Drawings

Figure 1 shows analgetic effect of the compound of the present invention on formalin model in rats.
Figure 2 shows analgetic effect of the compound of the present invention on Bennett hyperalgesia model in rats.

### Best Mode for Carrying Out the Invention

The following examples illustrate the present invention, but do not limit the present invention.

### Example 1: Antinoxious effect of intrathecal injection of the compound of the present invention in formalin test in rats.

### [methods]

Eight weeks old male Sprague-Dawley rats were used. According to the method of Yaksh et al., the rat was mounted on the brain stereotaxis apparatus under sodium pentobarbital anesthesia and a polyethylene tube filled with saline was threaded approximately 3cm into intraspinal side through the cisternal incision.

A day after implantation, the rat had no hindlimb paralysis was administered 50µl of 5% formalin subcutaneously and was placed in the observational plastic cage immediately. Formalin injection time was regarded as 0min and for 1min every 5min until 60min, number of leg-up response (flinch) was counted. The compound of formula (IA-1) ((+)-trans-3-imino-5-methyl-7-chloro-2-azabicyclo[4.1.0]heptane Monohydrochloride) of the present invention was dissolved in saline, subsequently the dose of 2 or 20µg/10µl/rat was administered intrathecally via polyethylene tube 10min prior to the formalin injection.

### [results]

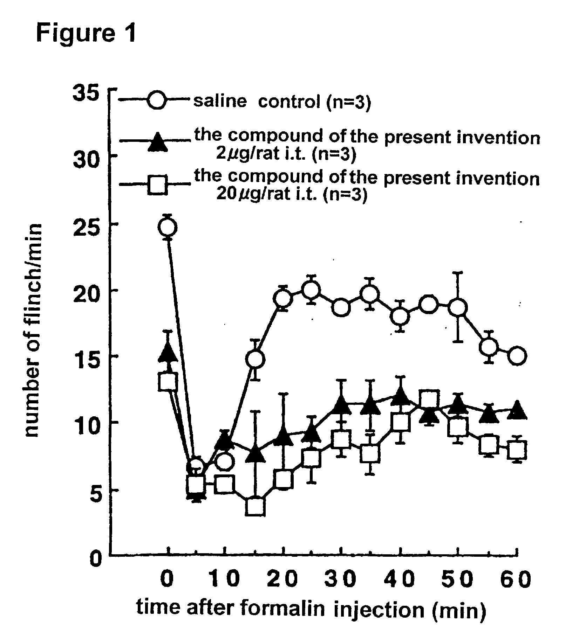

As shown in figure 1, number of flinch in the rats treated with both doses of the compound of the present invention was decreased in the first phase (0-10min after formalin injection) and the second phase (10-60min after formalin injection) compared to that in vehicle treated group.

### Example 2 Antinoxious effect of intrathecal injection of the compound of the present invention on hyperalgesia to thermostimulus in Bennett model in rats

### [methods]

Seven weeks old male Sprague-Dawley rats were used. According to the method of Yaksh et al. (*J.Physiol. Behav.* Vol.17, pp.1031-1036, 1976), the rat was mounted on the brain stereotaxis apparatus under sodium pentobarbital anesthesia and a polyethylene tube filled with saline was threaded about 3cm into intraspinal side through the cisternal incision.

A day after implantation, rats had no hindlimb paralysis were restrained in a prostrate position after induction of anesthetization with pentobarbital sodium. According to the method of Bennett and Xie et al. (*Pain*, Vol.33, pp.87-107, 1988), an incision of 2cm was cut along the skin of the femoral region of the right hindlimb, and the exposed sciatic nerve was constricted with 2 pieces of gut suture at 2 distal sites, and the incision was closed with a piece of silk suture. The left hindlimb was similarly operated on without performing any constriction of the sciatic nerve (sham-operated hindlimb).

Six days after the operation, the response latency to nociceptive thermostimulus was determined with the plantar test device, and rats with hyperalgesia were selected for the drug evaluation study on next day. Briefly, rat was placed in transparent acrylic cage installed with infrared rays from a halogenlamp illuminating from below the cage in a left-right crossover manner to target on the plantar side of hindlimb. By using a digital timer installed in the plantar testing device, the interval (response latency) from initiation of illumination to the time required for the animal to lift the hindlimb in an attempt to escape was measured. The nociceptive sensitivity was indexed by the difference in score (DS, expressed in second) derived from subtraction of the response latency of the sham-operated hindlimb from that of operated hindlimb. To select individuals with stabilized hyperalgesia by nociceptive thermostimulus, evaluation of the response latency was made. Rats exhibited stabilized hyperalgesia defined as DS of <-3.0 s in two consecutive examinations were used for the drug evaluation study on the next day. The compound of formula (IA-1) ((+)-trans-3-imino-5-methyl-7-chloro-2-azabicyclo[4.1.0]heptane Monohydrochloride) of the present invention was dissolved in saline, subsequently the dose of 0.2, 2 or 20µg/10µl/rat was administered intrathecally via polyethylene tube 20min prior to the planter test.

### [results]

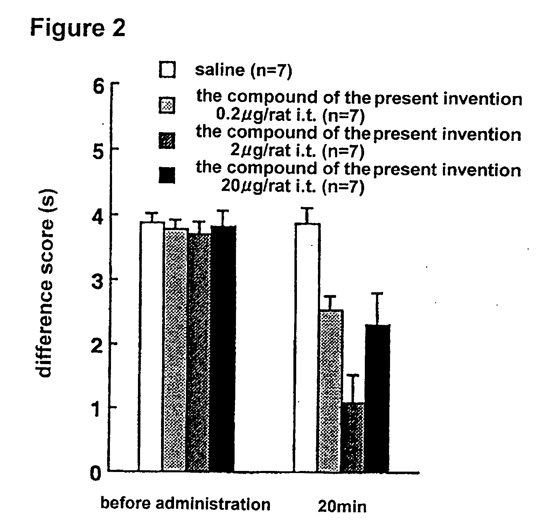

As shown in figure 2, the hyperalgesia to nociceptive thermostimulus was improved in rats treated with the compound of the present invention compared to vehicle treated rats.

### Formulation Example 1: Preparation of tablets

The following components were mixed and tableted in a conventional manner to obtain 100 tablets each containing 100 mg of the active ingredient.

| | |
|---|---|
| (+)-trans-3-imino-5-methyl-7-chloro-2-azabicyclo[4.1.0]heptane Monohydrochloride | 10g |
| Fibrin calcium glycolate (disintegrant) | 200mg |
| Magnesium stearate (lubricant) | 100mg |
| Microcrystalline cellulose | 9.7g |

### Formulation Example 2: Preparation of injection

(+)-trans-3-imino-5-methyl-7-chloro-2-azabicyclo[4.1.0]heptane Monohydrochloride (100mg) and mannitol (2000mg) were dissolved into distilled water (100ml), placed 10 ml portions into 50 ml ampoules and freeze-dried in a conventional manner to obtain 10 ampoules each containing 10 mg of active ingredient.

## Claims

1. A prevention and/or treatment of pain which comprise, as an active ingredient, a condensed piperidine compound represented by formula (I), an acid addition salt thereof or a hydrate thereof: 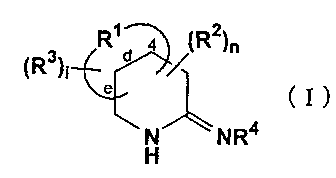 wherein -R1- represents a 3- or 4-membred carbocyclic ring together with the carbon atom or atoms to which it is bonded, the carbocyclic ring being condensed to side d or e of the piperidine ring or bonded to the 4-position of the piperidine ring through a spiro-bond;
R2 represents a C1-6 alkyl group;
R3 represents a C1-6 alkyl group, a C2-6 alkenyl group, a C2-6 alkynyl group or halogen atom;
R4 represents a hydrogen atom, an amino-C1-4 alkyl group or a carbocyclic ring-C1-4 alkyl group which may be substituted with an amino-C1-4 alkyl group;
i represents 0 or an integer of 1 to 3;
n represents 0 or an integer of 1 to 3; and
the plural R2's or R3's are the same or different.

2. The prevention and/or treatment of pain according to claim 1 which comprise, as an active ingredient, a condensed piperidine compound represented by formula (IA), an acid addition salt thereof or a hydrate thereof: 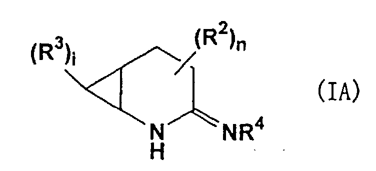 wherein all the symbols have the same meanings as defined in claim 1.

3. The prevention and/or treatment of pain according to claim 1 which comprise, as an active ingredient, a condensed piperidine compound represented by formula (I), an acid addition salt thereof or a hydrate thereof, wherein i is zero.

4. The prevention and/or treatment of pain according to claim 1 which comprise, as an active ingredient, a condensed piperidine compound represented by formula (I), an acid addition salt thereof or a hydrate thereof, wherein i is one, two or three, and R3 is selected from the group consisting of a C1-6 alkyl group, a C2-6 alkenyl group, and C2-6 alkynyl group.

5. The prevention and/or treatment of pain according to claim 1 which comprise, as an active ingredient, a condensed piperidine compound represented by formula (I), an acid addition salt thereof or a hydrate thereof, wherein i is one, two or three, and at least one of the plural R3's is a halogen atom.

6. The prevention and/or treatment of pain according to claim 1 which comprise, as an active ingredient, a condensed piperidine compound represented by formula (IA-1), an acid addition salt thereof or a hydrate thereof:

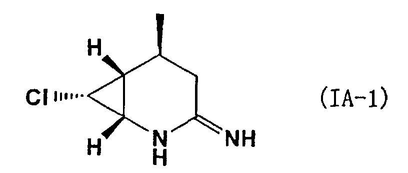

7. The prevention and/or treatment described in any one of claim 1 to 6 of pain, which is hyperalgesia.

8. The prevention and/or treatment described in any one of claim 1 to 6 of pain, which is allodynia.

9. The prevention and/or treatment described in any one of claim 1 to 8 of pain, which is allodynia with pain caused by peripheral or central nerve injury, diabetic neuropathy or nerve invasive cancer.
